# EUROPEAN PATENT APPLICATION

(11) **EP 1 553 080 A1**
(43) Date of publication of application: **13.07.2005**
(21) Application number: 03750738.1
(22) Date of filing: 26.09.2003
(51) Int. Cl.: C07C 233/61, C07C 229/08, A61K 31/164, A61P 35/00

(54) **USE OF CYCLIC DEPSIPEPTIDE AS A CHEMOTHERAPEUTIC AGENT AGAINST CANCER**

(30) Priority: 02.10.2002 ES 200202375
(71) Applicant: Universidad de Barcelona, 08028 Barcelona (ES)
(72) Inventor: PEREZ TOMAS, R., Centro de Patentes de la UB, 08028 Barcelona (ES); MONTANER RAMONEDA, B., Centro de Patentes de la UB, 08028 Barcelona (ES); GIRALT LLEDO, E., Centro de Patentes de la UB, 08028 Barcelona (ES); MARTINELL PEDEMONTE,M.,Centro de Patentes de la UB, 08028 Barcelona (ES); VILASECA CASAS, M., Centro de Patentes de la UB, 08028 08028 (ES)
(74) Representative: Barlocci, Anna
(86) International application number: PCT/ES2003/000489
(87) International publication number: WO 2004/031130

(57) **Abstract**

Serratamolide, also named cyclo[(3R)-3-hydroxydecanoyl-L-seryl-(3R)-3-hydroxydecanoyl-L-seryl], is a cyclic depsipeptide isolated from cultures of bacteria of the species Serratia marcescens. Serratamolide decreases the viability of, and induces apoptosis in several cancer cells (leukemias, myelomas, carcinomas, etc.), virtually with no effect on non-cancer cells. This makes serratamolide, and its pharmaceutically acceptable addition salts and/or solvates, useful as anticancer chemotherapeutic agent, particularly against leukemia, lymphoma, myeloma, carcinoma, melanoma and sarcoma.

## Description

This invention relates to the treatment of neoplasms or cancer by using a known cyclic depsipeptide of natural origin.

### BACKGROUND ART

Chemotherapy can cure certain forms of cancer. Many other forms of cancer benefit temporarily or partially from chemotherapy. Some cancers are resistant to drugs, however. As most cancer drugs are limited in their usefulness, there is a need for new chemotherapeutic anticancer agents.

One problem of cancer chemotherapy is that only a certain proportion of cells is dividing at any one time, and most drugs can destroy only that part of the cell population undergoing division. Another problem is that cancer drugs also damage normal cells and tissues. In addition, some cancer cells eventually become resistant to drugs.

Apoptosis, also called programmed cell death, is a mechanism that induces cells to self-destruct in response to the appropriate trigger. Apoptosis is initiated in various circumstances, such as when a cell is no longer needed within the body or when it becomes a threat to the health of the organism. Apoptosis is necessary in embryonic development and in the daily maintenance of a mature organism. The aberrant inhibition or initiation of apoptosis contributes to many diseases, including cancer. Apoptosis is distinguished from another form of cell death, called necrosis, which results from injury. Apoptosis is a normal physiological process that offsets cell proliferation. Cells in virtually all tissues may die through apoptosis for the good of the organism, including those cells which proliferate uncontrollably and form a cancerous tumor.

Apoptosis is involved in the action of several chemotherapeutic agents. In the last few years several new anticancer drugs based on apoptosis have been screened. They are expected to be effective against tumors with high proliferation like leukaemias or lung tumors, and also against those for which satisfactory treatment is not available, such as gastrointestinal tumors

Serratamolide (CAS RN 5285-25-6), also named cyclo[(3R)-3-hydroxydecanoyl-L-seryl-(3R)-3-hydroxy-decanoyl-L-seryl], is a cyclic depsipeptide having the formula shown below, with a specific stereochemistry in its four chiral centers. It was first isolated in 1961 from a Serratia marcenses culture. A total synthesis of serratamolide is also known (M.M. Shemyakin et al., Tetrahedron Lett. 1964, pp. 47-54).

It was found very early that serratamolide has activity against a number of bacteria, yeasts and pathogenic fungi (H.H. Wasserman et al., J. Am. Chem. Soc. 1961, vol. 83, p. 4107; 1962, vol. 84, p. 2978). More recently, some wetting/spreading and hemaglutinating activities of serratamolide have been reported (T. Matsuyama et al., J. Gen. Microbiol. 1986, vol. 132, p. 865; Y. Kawai et al., Eur. J. Biochem. 1988, vol. 171, p. 73). But the use of serratamolide as anticancer agent has neither been reported nor suggested.

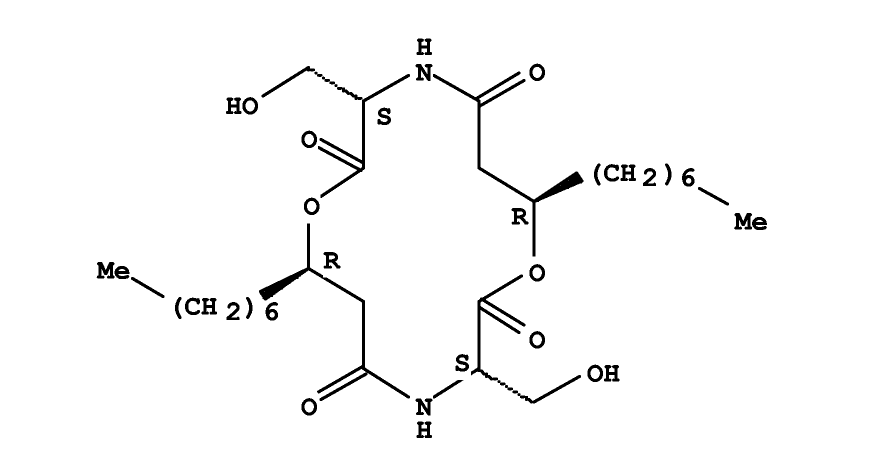

### SUMMARY OF THE INVENTION

As illustrated in the accompanying examples, it is disclosed here for the first time that serratamolide decreases the viability of, and induces apoptosis in several cancer cells (leukemias, myeloma, carcinomas, etc.), with virtually no effect on non-cancer cells.
This makes serratamolide useful as a chemotherapeutic anticancer agent.

Thus, one aspect of the present invention is the use of serratamolide -or of a pharmaceutically acceptable addition salt and/or solvate thereof- for the preparation of a medicament for the treatment and/or prophylaxis of cancer. In preferred embodiments, the cancer is selected from the group consisting of leukemia, lymphoma, myeloma, carcinoma, melanoma and sarcoma.

Serratamolide -or a pharmaceutically acceptable addition salt and/or solvate thereof- for the treatment and/or prophylaxis of cancer is also part of the present invention. Analogously, a pharmaceutical composition for the treatment and/or prophylaxis of cancer comprising a therapeutically effective amount of serratamolide -or of a pharmaceutically acceptable addition salt and/or solvate thereof- as active principle, together with pharmaceutically acceptable excipients or carriers, is also part of the invention.

Serratamolide can be isolated from a culture of bacteria of the species Serratia marcescens, as illustrated in the accompanying examples which should not be interpreted as a limitation of the invention.

### EXAMPLES

### Example 1: Isolation of serratamolide

Serratamolide was extracted by shaking bacterium Serratia marcescens 2170 cells (supplied by the Microbiology Department of the University of Barcelona) with a mixture of methanol and 1 N HCl (24:1). After centrifugation (6800 x g for 15 min), the solvent of the supernatant was evaporated under vacuum. Atmospheric pressure liquid chromatography of the extract was performed on silica gel with chloroform and methanol as solvents. The eluted pigmented fractions, containing two major products (further characterized as prodigiosin and serratamolide) were pooled and the chloroform/methanol extract was vacuum evaporated, redissolved in H₂O and lyophilized. The sample mixture was analyzed by electrospray ionization/mass spectrometry (ESI-MS), using a VG-Quattro® triple quadrupol mass spectrometer (Micromass, VG-Biotech, U.K.) and by matrix assisted laser desorption (MALDI) using a Voyager delayed extraction (DE) time of flight (TOF) mass spectrometer (Perseptive Biosystems, Framingham, USA). The product with the molecular weight data (m/z 515) consistent with the molecular mass expected for serratamolide was isolated from the pigmented mixture by two consecutive MPLC (medium pressure liquid chromatography) steps. MPLC was performed on a system provided with a CFG® Prominent/Duramat pump, a variable wavelength detector LKB® Bromma 2158 UVICORD SD (206 nm filter), a Gilson collector FC 205 and a Pharmacia-LKB REC 101 register. In the first step a Lichroprep C₈ reversed-phase glass column (20 x 4 cm) was used with a 0-100% B linear gradient (A: 0.01 M ammonium acetate pH 7, B: 100% acetonitrile), 1000 ml total volume at a flow rate 240 ml/h. The eluted fractions were analyzed by HPLC (high performance liquid chromatography) on a Shimadzu instrument LC-6A with a Nucleosil® C₁₈ analytical reversed-phase column (25 x 0.4 cm) and by MALDI-TOF. Fractions containing serratamolide were pooled, vacuum evaporated and lyophilized. The second MPLC purification step was performed using a C₁₈ reversed-phase glass column (26 x 2.5 cm) and a 35-55 % B linear gradient (A: H₂O 0.045 % trifluoroacetic acid, B: CH₃CN 0.036 % trifluoroacetic acid), 800 ml total volume at a flow rate 120 ml/h. The eluted fractions were analyzed by MALDI-TOF and HPLC. 6.6 mg of product (96 % purity, determined by HPLC) was obtained after pooling, vacuum evaporation and lyophilisation of the desired fractions. Quantification was performed by amino acid analysis on a Beckman 6300 automatic analyzer with a sulfonated-polystyrene column (25 x 0.4 cm, Beckman 7300/6300). The product was characterized by mass spectrometry, nuclear magnetic resonance (NMR) spectroscopy and gas chromatography (GC).
Mass spectrometry: Exact mass determination by chemical ionization (CI), (with methane in an AutoSpec magnetic sector mass spectrometer N/S-X134): m/z 515.335020 (M+H)⁺. ESI-MS: m/z 515.2 (M+H)⁺. MALDI-TOF: m/z 515.8 (M+H)⁺, 537.8 (M+Na)⁺, 553.8 (M+K)⁺. The molecular weight data was consistent with the molecular formula of serratamolide: C₂₆H₄₆N₂O₈ (MW monoisotopic: 514.3254, average: 514.6599).
NMR spectroscopy: NMR spectra were recorded on either a Varian Inova-500 or a Bruker DMX-500 spectrometers, operating at 500 MHz and 125 MHz for ¹H and ¹³C respectively. Spectra were recorded in CD₃OH or CD₃OD at 298K. The assignment of the NMR signals were carried out using a combination of ¹H-1D; ¹H-COSY; ¹³C-1D; ¹³C-DEPT and ¹H-¹³C-HSQC experiments. The results were in agreement with previous NMR studies (cf. C.H. Hassall et al., J. Chem. Soc. (B), 1971, pp. 1757-61).

### Gas Chromatography:

(a) Acid Hydrolysis of serratamolide.- Serratamolide (1.5 mg) was heated with 0.5 ml HCl 6 N at 110 °C for 30 h. The sample was evaporated to dryness with clean, dry nitrogen. Serratamolide hydrolysis products in these conditions yielded 2 eq. serine and 2 eq. 3-hydroxydecanoic acid. The serine stereocenter was determined by derivatization of this aminoacid as N-pentafluoropropionic isopropyl ester and analysis of the crude product by CG on a chiral column. Coinjection of the sample with derivatized L-serine and DL-serine references was performed.
(b) Derivatization of serine as N-PFP isopropyl ester.-0.5 ml of 2 N HCl in isopropyl alcohol were added to the dried sample and heated at 100 °C for 1 h. The reaction mixture was evaporated to dryness with clean, dry nitrogen. 0.5 ml ethyl acetate and 50 µl of pentafluoropropionic anhydride (PFPA) were added, and the mixture heated at 100 °C for 30 min. Evaporation to dryness was again performed with dry nitrogen and the residue was dissolved in 10 µl methylene chloride and analyzed by CG.
(c) Separation.- GC Conditions for D-and L-isomers separation of Serine derivatives (and aminoacids in general) were as follows: 50 m CHIRASIL-VAL-III column (Alltech catalog no. 13137). Temperature gradient: 80 °C (3 min)-190 °C at 4 °C/min. A HP 5890 gas chromatograph was used. One major peak was obtained in the chromatographic analysis of hydrolyzed and derivatized serratamolide which corresponded in retention time to the L-serine derivative. No peak corresponding to D-serine was observed.

Conformational studies of Serratamolide were carried out using molecular dynamics. Considering the NMR data and the serine's configuration, the results were only consistent with an (R)-3-hydroxydecanoic acid.

### Example 2: Activity of serratamolide against the viability of cancer cells

The following cancer cell lines were used:
- Jurkat clone E6-1: Acute human T cell leukaemia cells.
- Molt-4: peripheral blood, acute human lymphoblastic leukaemia.
- NSO: human myeloma cells.
- GLC-4S: human small lung carcinoma cells.
- HGT-1: human gastric carcinoma cells.
- HT-29: human colon adenocarcinoma cells.
The following nonmalignant cells lines were used for comparative purposes:
- NIH-3T3: Swiss mouse embryo cells.
- NRK-49F: normal rat kidney cells.
- IEC-18: normal epithelial cells of rat ileum.

The effect of serratamolide on the viability of different cancer cell lines (Jurkat, Molt-4, NSO, HGT-1, HT-29 and GLC-4S) and nonmalignant cell lines (NIH-3T3, NRK-49F and IEC-18) was determined by the MTT assay (cf. J. Mosmann, J. Immunol. Meth. 1983, vol. 65, pp. 55-63), MTT standing for 3-(4,5-dimethylthiazol-2-yl)-2,5-diphenyltetrazolium bromide, thiazole blue. In this assay, 20x10³ cells were incubated in 96-well microtiter cell culture plates, in the absence (control cells) or in the presence of increasing amounts of serratamolide, ranging from 5 to 40 µM, in a final volume of 100 µl. After 24 h incubation, 10 µM of MTT was added to each well for an additional 4 hours. The blue MTT formazan precipitate was dissolved in 100 µl of isopropanol: 1 N HCl (24:1) and the absorbance at 550 nm was measured on a multiwell plate reader. Cell viability was expressed as a percentage of control.
A dose-dependent decrease in the number of viable cells was observed in all the cancer cell lines studied whereas we not observe a significant decrease in the viability of the nonmalignant cell lines. The IC₅₀ was 15.0 µM for Jurkat, 12.2 µM for Molt-4, 17.1 µM for NSO, 17.3 µM for GLC-4S, 25.8 µM for HGT-1 and 38.9 µM for HT-29. However NRK-49F didn't shown reduction in the number of viable cells in presence of 30 µM of serratamolide and 85 % of NIH-3T3 cells and 75 % of IEC-18 were viable in the presence at the same dose.

### Example 3: Serratamolide induction of apoptosis in cancer cells

In order to determine whether the observed cytotoxic effect was due to apoptosis, it was analyzed whether serratamolide induces DNA fragmentation by agarose gel electrophoresis. In the assay, 10⁶ cells/ml were exposed to 20 µM serratamolide and incubated overnight (16 h). Cells were washed in PBS (phosphate buffer saline) and resuspended in ice-cold lysis buffer (10 mM Tris-HCl pH 7.4, 1 mM EDTA, 0.2 % Triton X-100). After incubation for 15 minutes at 4 °C, cell lysates were centrifuged at 14,000 x g for 15 minutes to separate low molecular weight DNA from intact chromatin. The supernatant was treated with 0.2 mg/ml of proteinase K in a buffer containing 150 mM NaCl, 10 mM Tris-HCl pH 8.0, 40 mM EDTA and 1 % SDS (sodium dodecyl sulfate) for 4 h at 37 °C. The DNA preparations were phenol/chloroform extracted twice to remove proteins. DNA was precipitated with 140 mM NaCl and two volumes of ethanol at -20 °C overnight. DNA precipitates were recovered by centrifugation at 14,000 x g for 10 minutes at 4 °C, washed twice in cool 70 % ethanol and air dried. DNA pellets were resuspended in 15 µl of TE (10 mM Tris-HCl pH 8.0, 1 mM EDTA) and treated with DNase-free RNase (Boehringer Mannheim) for 1 h at 37 °C. 3.2 µl of loading buffer was added to each tube and the DNA preparations were electrophoresed in 1 % agarose gels which contained ethidium bromide. Gels were placed on an UV light box to visualize the DNA ladder pattern. Agarose gel electrophoresis of DNA showed the characteristic ladder pattern of apoptosis in the Jurkat haematopoietic cancer cell line. However, DNA laddering was not observed in the NIH-3T3 nonmalignant cells. Furthermore, the characteristics apoptotic bodies were observed in Jurkat cells after treatment with serratamolide, while they were not observed in NRK-49F treated cells.

## Claims

1. Serratamolide, or a pharmaceutically acceptable addition salt and/or solvate thereof, for the treatment and/or prophylaxis of cancer.

2. Product according to claim 1 wherein the cancer is selected from the group consisting of leukemia, lymphoma, myeloma, carcinoma, melanoma and sarcoma.

3. Use of serratamolide, or of a pharmaceutically acceptable addition salt and/or solvate thereof, for the preparation of a medicament for the treatment and/or prophylaxis of cancer.

4. Use according to claim 3 wherein the cancer is selected from the group consisting of leukemia, lymphoma, myeloma, carcinoma, melanoma and sarcoma.

5. A pharmaceutical composition for the treatment and/or prophylaxis of cancer, comprising a therapeutically effective amount of serratamolide, or of a pharmaceutically acceptable addition salt and/or solvate thereof, as active principle, together with pharmaceutically acceptable excipients or carriers.

6. The composition according to claim 5 wherein the cancer is selected from the group consisting of leukemia, lymphoma, myeloma, carcinoma, melanoma and sarcoma.
